# EUROPEAN PATENT APPLICATION

(11) **EP 4 775 564 A1**
(43) Date of publication of application: **15.07.2026**
(21) Application number: 24862399.3
(22) Date of filing: 03.06.2024
(51) Int. Cl.: C07C 69/653, A61K 6/30, A61K 49/04

(54) **X-RAY RADIOPAQUE COMPOUND AND DENTAL ADHESIVE COMPOSITION**

(30) Priority: 06.09.2023 JP 2023144346
(71) Applicant: GC R&D Corporation, Tokyo 174-8585 (JP)
(72) Inventor: SATO, Yuna, Tokyo 174-8585 (JP); OHARA, Yuki, Tokyo 174-8585 (JP); SHOJI, Takumi, Tokyo 174-8585 (JP); MINAMISAWA, Hiroto, Tokyo 174-8585 (JP)
(74) Representative: Müller-Boré & Partner Patentanwälte PartG mbB
(86) International application number: PCT/JP2024/020224
(87) International publication number: WO 2025/052742

(57) **Abstract**

A radiopaque compound includes a structural moiety A including a halogen atom and having an acyclic structure; and a structural moiety B derived from a (meth)acrylate.

## Description

### TECHNICAL FIELD

The present invention relates to a radiopaque compound, and a dental adhesive composition.

### BACKGROUND ART

Radiopacity is required for dental adhesive compositions in the field of dentistry from the viewpoint of preventing misdiagnosis of secondary caries. Generally, compounds derived from polymerizable monomers or polymerizable polymers included in dental adhesive compositions do not exhibit radiopacity. Therefore, dental adhesive compositions including a filler, such as barium or the like, for impartment of radiopacity are widely used.

The filler reacts with, and inactivates, acidic monomers included in the dental adhesive compositions. Thus, in recent years, there is a need for a filler-free dental adhesive composition in which radiopacity is imparted to a polymerizable monomer or a compound derived from a polymerizable polymer.

For example, a brominated polymerizable monomer including: a resin matrix having a specific structure with radiopacity; a filler matrix in an amount of at least 25% by weight relative to the weight of a dental composition; and an initiator, is proposed (see, for example, Patent Literature 1).

### CITATION LIST

### PATENT LITERATURE

Patent Literature 1: PCT Japanese Translation Patent Publication No. 2021-502357

### SUMMARY OF THE INVENTION

### TECHNICAL PROBLEM

However, in conventional dental adhesive compositions including the invention described in Patent Literature 1, a halogen with radiopacity is bonded to the benzene ring in an aromatic compound included in the dental adhesive compositions. Thus, there is a concern that release of the halogen easily occurs in terms of the structure, resulting in reduction in radiopacity. Also, these radiopaque compounds have a problem that their synthesis routes are complicated.

In view of the problems and concerns in the conventional technique, it is an object of the present invention to provide a radiopaque compound that has excellent radiopacity, can have suppressed release of a halogen, and can be simply synthesized.

### SOLUTION TO THE PROBLEM

A radiopaque compound of the present invention as a means for solving the problems includes: a structural moiety A including a halogen atom and having an acyclic structure; and a structural moiety B derived from a (meth)acrylate.

### ADVANTAGEOUS EFFECTS OF THE INVENTION

According to the present invention, it is possible to provide a radiopaque compound that has excellent radiopacity, can have suppressed release of a halogen, and can be simply synthesized.

### DETAILED DESCRIPTION OF THE INVENTION

Hereinafter, the present invention will be described in detail.

### (Radiopaque Compound)

The radiopaque compound of the present invention includes a structural moiety A including a halogen atom and having an acyclic structure, and a structural moiety B derived from (meth)acrylate, and may include other structural moieties, if necessary.

The other structural moieties may be appropriately selected as long as the effects of the present invention are not impaired.

In the present specification, "structural moiety A including a halogen atom and having an acyclic structure" may be referred to simply as "structural moiety A", and "structural moiety B derived from a (meth)acrylate" may be referred to simply as "structural moiety B".

In the present specification, "radiopaque compound" may be referred to simply as "compound".

In the present specification, "(meth)acrylate" refers to one or both of an acrylate and a methacrylate.

As described above, the radiopaque compound can have suppressed release of the halogen. In other words, the radiopaque compound can have suppressed release of the halogen to maintain high radiopacity. In the present invention, although a mechanism by which the release of the halogen is suppressed is not clearly found, the suppression of the release of the halogen is presumed to be due to the structural stability of the radiopaque compound.

Also, the radiopaque compound has X-ray opacity (the property of blocking irradiated X-rays without transmitting the X-rays) and thus has enhanced radiopacity.

### <Structural Moiety A>

No particular limitation is imposed on the structural moiety A as long as the structural moiety A is a structural moiety including a halogen atom and derived from an acyclic structure. The structural moiety A may be appropriately selected in accordance with the intended purpose.

No particular limitation is imposed on the acyclic structure as long as the acyclic structure does not have a cyclic structure. The acyclic structure may be appropriately selected in accordance with the intended purpose, and is, for example, a saturated or unsaturated acyclic hydrocarbon structure.

The acyclic structure is preferably a branched chain, and more preferably includes a halogen atom in a side chain. In the present specification, "main chain" indicates the longest linear chain in the compound including the structural moiety A and the structural moiety B, and "side chain" indicates a molecular chain that is not the main chain. The "main chain" in the structural moiety A indicates a molecular chain included in the main chain of the compound, and the "side chain" in the structural moiety A indicates a molecular chain that is not the main chain. In the structural moiety A, when the lengths of the branched chains are the same, the molecular chain including a halogen atom is defined as the side chain.

No particular limitation is imposed on the structural moiety A as long as the structural moiety A includes a halogen atom and has an acyclic structure. The structural moiety A may be appropriately selected in accordance with the intended purpose. However, from the viewpoint of simplifying the synthesis of the compound, the structural moiety A preferably includes a hydroxy group, an isocyanate group, an epoxy group, an amino group, or a carboxyl group, and more preferably includes a hydroxy group.

### <Structural Moiety B>

No particular limitation is imposed on the structural moiety B as long as the structural moiety B is a structural moiety derived from a (meth)acrylate. The structural moiety B may be appropriately selected in accordance with the intended purpose.

No particular limitation is imposed on the (meth)acrylate, and the (meth)acrylate may be appropriately selected in accordance with the intended purpose. However, from the viewpoint of simplifying the synthesis of the compound, the (meth)acrylate preferably includes a hydroxy group, an isocyanate group, an epoxy group, an amino group, or a carboxyl group, and more preferably includes a carboxylic acid group.

No particular limitation is imposed on the (meth)acrylate including a carboxylic acid group. The (meth)acrylate including a carboxylic acid group may be appropriately selected in accordance with the intended purpose, and is, for example, mono(2-acryloyloxyethyl) succinate, 2-carboxyethyl acrylate, or the like.

The (meth)acrylate may be an appropriately synthesized product or a commercially available product. Examples of the commercially available product of the (meth)acrylate include, as product names, mono(2-acryloyloxyethyl) succinate (available from TOKYO CHEMICAL INDUSTRY CO., LTD.), 2-carboxyethyl acrylate (available from TOKYO CHEMICAL INDUSTRY CO., LTD.), and the like.

The radiopaque compound preferably includes at least one structure selected from a structure represented by general formula (1) below, a structure represented by general formula (2) below, or a structure represented by general formula (3) below. 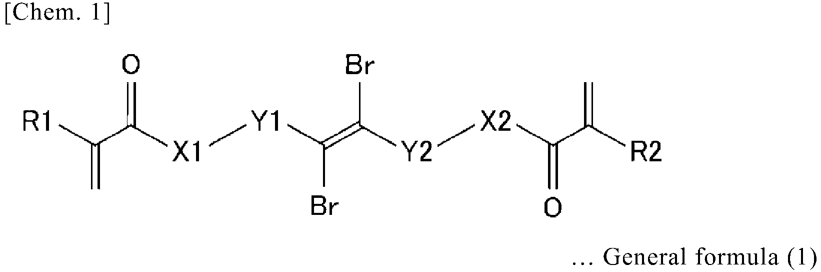 (In the general formula (1), R1 and R2 each independently represent "-H" or "-CH₃", X1 and X2 each independently represent "-O-" or "-NH-", and Y1 and Y2 each independently represent a linear or branched hydrocarbon group having 1 or more and 20 or less carbon atoms, which optionally includes an ether bond, an ester bond, and/or an amide bond.) 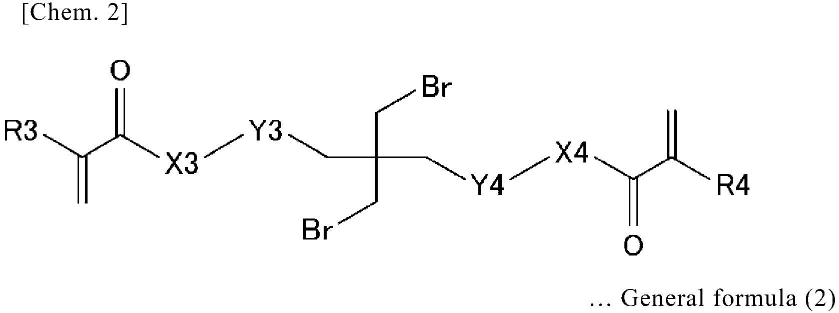 (In the general formula (2), R3 and R4 each independently represent "-H" or "-CH₃", X3 and X4 each independently represent "-O-" or "-NH-", and Y3 and Y4 each independently represent a linear or branched hydrocarbon group having 1 or more and 20 or less carbon atoms, which optionally includes an ether bond, an ester bond, and/or an amide bond.) 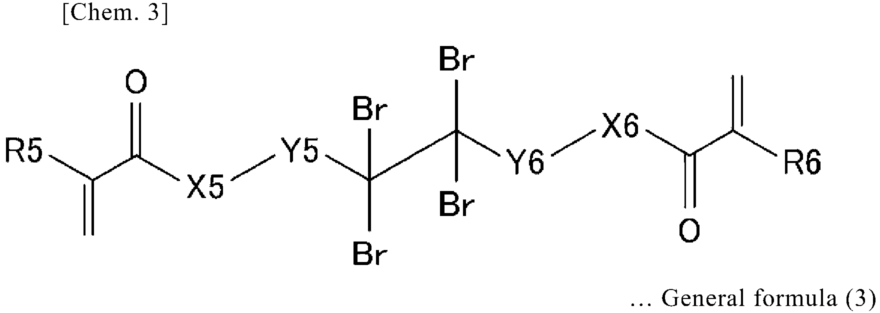 (In the general formula (3), R5 and R6 each independently represent "-H" or "-CH₃", X5 and X6 each independently represent "-O-" or "-NH-", and Y5 and Y6 each independently represent a linear or branched hydrocarbon group having 1 or more and 20 or less carbon atoms, which optionally includes an ether bond, an ester bond, and/or an amide bond.)

No particular limitation is imposed on the method of obtaining the structure represented by the general formula (1). The structure represented by the general formula (1) may be appropriately synthesized. Alternatively, a commercially available product including the structure represented by the general formula (1) may be used.

No particular limitation is imposed on the commercially available product including the structure represented by the general formula (1). The commercially available product including the structure represented by the general formula (1) may be appropriately selected in accordance with the intended purpose, and is, for example, trans-2,3-dibromo-2-butene-1,4-diol (available from TOKYO CHEMICAL INDUSTRY CO., LTD.).

No particular limitation is imposed on the method of obtaining the structure represented by the general formula (2). The structure represented by the general formula (2) may be appropriately synthesized. Alternatively, a commercially available product including the structure represented by the general formula (2) may be used.

No particular limitation is imposed on the commercially available product including the structure represented by the general formula (2). The commercially available product including the structure represented by the general formula (2) may be appropriately selected in accordance with the intended purpose, and is, for example, 2,2-bis(bromomethyl)-1,3-propanediol (available from TOKYO CHEMICAL INDUSTRY CO., LTD.).

No particular limitation is imposed on the method of obtaining the structure represented by the general formula (3). The structure represented by the general formula (3) may be appropriately synthesized. Alternatively, a commercially available product including the structure represented by the general formula (3) may be used.

The structure represented by the general formula (3) can be, for example, synthesized as 2,2,3,3-tetrabromobutane-1,4-diol (TBBD) by adding Br₂ into the triple bond of 2-butyne-1,4-diol.

No particular limitation is imposed on the synthesis method of the radiopaque compound. For example, the radiopaque compound may be synthesized by the following method.

### [Example of Synthesis Method of Radiopaque Compound]

Trans-2,3-dibromo-2-butene-1,4-diol, mono(2-acryloyloxyethyl) succinate, and a solution (e.g., water, ethyl acetate, or toluene) are heated overnight under reflux. Here, if necessary, a catalyst (e.g., concentrated sulfuric acid or tosic acid) and a condensing agent (e.g., N,N'-dicyclohexylcarbodiimide (DCC)) may be added. Also, if necessary, the synthesis may be performed under a protective gas atmosphere (e.g. nitrogen).

Next, the reaction product is cooled to room temperature, and then the reaction product is extracted from the solution. Subsequently, the reaction product is washed with water at least once, and dried over anhydrous Na₂SO₄ to remove the solvent in vacuum.

No particular limitation is imposed on the weight average molecular weight of the radiopaque compound, and the weight average molecular weight of the radiopaque compound may be appropriately selected in accordance with the intended purpose. The weight average molecular weight of the radiopaque compound is preferably 100 or more and 5,000 or less, and more preferably 200 or more and 1,000 or less.

When the weight average molecular weight of the radiopaque compound is in the above numerical range, the radiopaque compound and other components described below can be successfully mixed in the preparation of the dental adhesive composition described below. This is preferable because the dental adhesive composition described below can be easily prepared, the operability in use of the dental adhesive composition can be improved, and suitable adhesiveness can be ensured.

### (Dental Adhesive Composition)

The dental adhesive composition of the present invention includes the radiopaque compound including the structural moiety A including the halogen atom and having the acyclic structure, and the structural moiety B derived from the (meth)acrylate, and may include other components, if necessary.

Since the radiopaque compound is the same as that described in the section (Radiopaque Compound) described above, duplicate description will be omitted.

No particular limitation is imposed on the amount of the radiopaque compound. The amount of the radiopaque compound may be appropriately selected in accordance with the intended purpose, and is preferably 0.5% by mass or more and 70% by mass or less, more preferably 5% by mass or more and 60% by mass or less, and further preferably 15% by mass or more and 50% by mass or less, relative to the total amount of the dental adhesive composition.

The amount of the radiopaque compound is preferably 0.5% by mass or more relative to the total amount of the dental adhesive composition because the radiopacity in a cured product of the dental adhesive composition is enhanced.

The amount of the radiopaque compound is preferably 70% by mass or less relative to the total amount of the dental adhesive composition because the other components described below can be included in sufficient amounts, and various performances as the dental adhesive composition can be enhanced.

### <Other Components>

No particular limitation is imposed on the other components as long as the effects of the present invention are not impaired. The other components can be selected in accordance with the intended purpose. Examples of the other components include acid group-including (meth)acrylates, acid group-free (meth)acrylates, polymerization initiators, polymerization inhibitors, fillers, solvents, and the like.

### <<Acid Group-including (Meth)acrylates>>

Examples of the acid group in the acid group-including (meth)acrylates include a phosphoric acid group, a pyrophosphoric acid group, a thiophosphoric acid group, a sulfonic acid group, a phosphonic acid group, a carboxylic acid group, and the like.

Of these, phosphoric acid group-, thiophosphoric acid group-, or carboxylic acid group-including (meth)acrylates are preferable from the viewpoints of solubility of the smear layer on the tooth surface and tooth decalcification exhibited by the dental adhesive composition, especially adhesiveness to the enamel. The acid group-including (meth)acrylate may include a plurality of acid groups.

### -Phosphoric Acid Group-including (Meth)acrylates-

No particular limitation is imposed on the phosphoric acid group-including (meth)acrylates, and the phosphoric acid group-including (meth)acrylates may be appropriately selected in accordance with the intended purpose. Examples of the phosphoric acid group-including (meth)acrylates include 2-(meth)acryloyloxyethyl dihydrogen phosphate, bis[2-(meth)acryloyloxyethyl] hydrogen phosphate, 2-(meth)acryloyloxyethylphenyl hydrogen phosphate, 6-(meth)acryloyloxyhexyl dihydrogen phosphate, 6-(meth)acryloyloxyhexylphenyl hydrogen phosphate, 10-(meth)acryloyloxydecyl dihydrogen phosphate (MDP), 1,3-di(meth)acryloylpropan-2-dihydrogen phosphate, 1,3-di(meth)acryloylpropan-2-phenylhydrogen phosphate, bis[5-{2-(meth)acryloyloxyethoxycarbonyl}heptyl] hydrogen phosphate, and the like.

These may be used alone or in combination.

### -Pyrophosphoric Acid Group-including (Meth)acrylates-

No particular limitation is imposed on the pyrophosphoric acid group-including (meth)acrylates, and the pyrophosphoric acid group-including (meth)acrylates may be appropriately selected in accordance with the intended purpose. Examples of the pyrophosphoric acid group-including (meth)acrylates include bis[2-(meth)acryloyloxyethyl] pyrophosphate, bis[4-(meth)acryloyloxybutyl] pyrophosphate, bis[6-(meth)acryloyloxyhexyl] pyrophosphate, bis[8-(meth)acryloyloxyoctyl] pyrophosphate, bis[10-(meth)acryloyloxydecyl] pyrophosphate, and the like.

These may be used alone or in combination.

### -Thiophosphoric Acid Group-including (Meth)acrylates-

No particular limitation is imposed on the thiophosphoric acid group-including (meth)acrylates, and the thiophosphoric acid group-including (meth)acrylates may be appropriately selected in accordance with the intended purpose. Examples of the thiophosphoric acid group-including (meth)acrylates include 2-(meth)acryloyloxyethyl dihydrogen thiophosphate, 3-(meth)acryloyloxypropyl dihydrogen thiophosphate, 4-(meth)acryloyloxybutyl dihydrogen thiophosphate, 5-(meth)acryloyloxypentyl dihydrogen thiophosphate, 6-(meth)acryloyloxyhexyl dihydrogen thiophosphate, 7-(meth)acryloyloxyheptyl dihydrogen thiophosphate, 8-(meth)acryloyloxyoctyl dihydrogen thiophosphate, 9-(meth)acryloyloxynonyl dihydrogen thiophosphate, 10-(meth)acryloyloxydecyl dihydrogen thiophosphate (MDTP), 11-(meth)acryloyloxyundecyl dihydrogen thiophosphate, 12-(meth)acryloyloxydodecyl dihydrogen thiophosphate, 13-(meth)acryloyloxytridecyl dihydrogen thiophosphate, 14-(meth)acryloyloxytetradecyl dihydrogen thiophosphate, 15-(meth)acryloyloxypentadecyl dihydrogen thiophosphate, 16-(meth)acryloyloxyhexadecyl dihydrogen thiophosphate, 17-(meth)acryloyloxyheptadecyl dihydrogen thiophosphate, 18-(meth)acryloyloxyoctadecyl dihydrogen thiophosphate, 19-(meth)acryloyloxynonadecyl dihydrogen thiophosphate, 20-(meth)acryloyloxyicosyl dihydrogen thiophosphate, and the like.

These may be used alone or in combination.

### -Carboxylic Acid Group-including (Meth)acrylates-

No particular limitation is imposed on the carboxylic acid group-including (meth)acrylates, and the carboxylic acid group-including (meth)acrylates may be appropriately selected in accordance with the intended purpose. Examples of the carboxylic acid group-including (meth)acrylates include 2-methacryloyloxyethyl succinate, 4-(meth)acryloyloxyethyl trimellitate, 4-(meth)acryloyloxyethyl trimellitic anhydride, 4-(meth)acryloyloxydecyl trimellitate, 4-(meth)acryloyloxydecyl trimellitic anhydride, 11-(meth)acryloyloxy-1,1-undecanedicarboxylate, 1,4-di(meth)acryloyloxy pyromellitate, 2-(meth)acryloyloxyethyl maleate, 2-(meth)acryloyloxyethyl phthalate, 2-(meth)acryloyloxyethyl hexahydrophthalate, and the like.

These may be used alone or in combination.

### -Sulfonic Acid Group-including (Meth)acrylates-

No particular limitation is imposed on the sulfonic acid group-including (meth)acrylates, and the sulfonic acid group-including (meth)acrylates may be appropriately selected in accordance with the intended purpose. Examples of the sulfonic acid group-including (meth)acrylates include 2-(meth)acrylamide-2-methylpropanesulfonate, styrenesulfonate, 2-sulfoethyl (meth)acrylate, and the like.

These may be used alone or in combination.

### -Phosphonic Acid Group-including (Meth)acrylates-

No particular limitation is imposed on the phosphonic acid group-including (meth)acrylates, and the phosphonic acid group-including (meth)acrylates may be appropriately selected in accordance with the intended purpose. Examples of the phosphonic acid group-including (meth)acrylates include 2-(meth)acryloyloxyethyl phenyl phosphonate, 5-(meth)acryloyloxypentyl-3-phosphonopropionate, 6-(meth)acryloyloxyhexyl-3-phosphonopropionate, 10-(meth)acryloyloxydecyl-3-phosphonopropionate, 6-(meth)acryloyloxyhexyl-3-phosphonoacetate, 10-(meth)acryloyloxydecyl-3-phosphonoacetate, and the like.

These may be used alone or in combination.

These acid group-including (meth)acrylates may be used alone or in combination.

Of these, from the viewpoint of enhancing adhesiveness in a cured product of the dental adhesive composition, 10-(meth)acryloyloxydecyl dihydrogen phosphate (MDP), 10-(meth)acryloyloxydecyl dihydrogen thiophosphate (MDTP), and 4-(meth)acryloyloxydecyl trimellitic anhydride (4-META) are preferable.

No particular limitation is imposed on the amount of the acid group-including (meth)acrylate, and the amount of the acid group-including (meth)acrylate may be appropriately selected in accordance with the intended purpose. The amount of the acid group-including (meth)acrylate is preferably 0.1% by mass or more and 40% by mass or less, more preferably 1% by mass or more and 25% by mass or less, and more preferably 5% by mass or more and 25% by mass or less, relative to the total amount of the dental adhesive composition.

The amount of the acid group-including (meth)acrylate is preferably 0.1% by mass or more relative to the total amount of the dental adhesive composition because the decalcification force of the dental adhesive composition to the tooth substance is further increased.

The amount of the acid group-including (meth)acrylate is preferably 40% by mass or less relative to the total amount of the dental adhesive composition because the curability of the dental adhesive composition is enhanced.

### <<Acid Group-free (Meth)acrylates>>

No particular limitation is imposed on the acid group-free (meth)acrylates, and the acid group-free (meth)acrylates may be appropriately selected in accordance with the intended purpose. Examples of the acid group-free (meth)acrylates include methyl (meth)acrylate, ethyl (meth)acrylate, isopropyl (meth)acrylate, n-butyl (meth)acrylate, isobutyl (meth)acrylate, hydroxypropyl (meth)acrylate, tetrahydrofurfuryl (meth)acrylate, glycidyl (meth)acrylate, 2-hydroxyethyl (meth)acrylate, 2-hydroxypropyl (meth)acrylate, 3-hydroxypropyl (meth)acrylate, 2-methoxyethyl (meth)acrylate, 2-ethoxyethyl (meth)acrylate, 2-methylhexyl (meth)acrylate, 2-ethylhexyl (meth)acrylate, benzyl (meth)acrylate, 2-hydroxy-1,3-di(meth)acryloyloxypropane, ethylene glycol di(meth)acrylate, diethylene glycol di(meth)acrylate, triethylene glycol di(meth)acrylate, butylene glycol di(meth)acrylate, neopentyl glycol di(meth)acrylate, 1,3-butanediol di(meth)acrylate, 1,4-butanediol di(meth)acrylate, 1,6-hexanediol di(meth)acrylate, trimethylolpropane tri(meth)acrylate, trimethylolethane tri(meth)acrylate, pentaerythritol tri(meth)acrylate, trimethylolmethane tri(meth)acrylate, pentaerythritol tetra(meth)acrylate, polybutylene glycol di(meth)acrylate, bisphenol A diglycidyl (meth)acrylate, di-2-(meth)acryloyloxyethyl-2,2,4-trimethylhexamethylene dicarbamate, 1,3,5-tris[1,3-bisi(meth)acryloyloxyl-2-propoxycarbonylaminohexane]-1,3,5-(1H,3H,5H)triazine-2,4,6-trione, 2,2-bis[4-(3-(meth)acryloyloxy-2-hydroxypropyl)phenyl]propane, N,N'-(2,2,4-trimethylhexamethylene)bis[2-(aminocarboxy)propane-1,3-diol]tetramethacrylate, and the like.

The acid group-free (meth)acrylates may be used alone or in combination.

Of these, from the viewpoints of increasing the mechanical strength in the cured product of the dental polymerizable composition and of enhancing adhesiveness to ceramics and the tooth substance, 2-hydroxy-1,3-di(meth)acryloyloxypropane (GDMA) and triethylene glycol di(meth)acrylate (TEGDMA) are preferable.

No particular limitation is imposed on the amount of the acid group-free (meth)acrylate, and the amount of the acid group-free (meth)acrylate may be appropriately selected in accordance with the intended purpose. The amount of the acid group-free (meth)acrylate is preferably 3% by mass or more and 50% by mass or less, more preferably 5% by mass or more and 40% by mass or less, and more preferably 10% by mass or more and 30% by mass or less, relative to the total amount of the dental adhesive composition.

The amount of the acid group-free (meth)acrylate is preferably 3% by mass or more relative to the total amount of the dental adhesive composition because the operability of the dental adhesive composition can be improved.

The amount of the acid group-free (meth)acrylate is preferably 50% by mass or less relative to the total amount of the dental adhesive composition because the mechanical strength of the cured product of the dental adhesive composition can be increased.

### <<Polymerization Initiator>>

No particular limitation is imposed on the polymerization initiator, and the polymerization initiator may be appropriately selected in accordance with the intended purpose. Examples of the polymerization initiator include chemical polymerization initiators, photopolymerization initiators, and the like. Of these, photopolymerization initiators are preferable because the dental adhesive composition is often cured by irradiation with visible light or the like.

Examples of the chemical polymerization initiators include thiourea derivatives, vanadium compounds, tertiary amines, organic peroxides, and the like.

### -Thiourea Derivatives-

Of the chemical polymerization initiators, the thiourea derivatives function as a reducing agent.

No particular limitation is imposed on the thiourea derivatives, and the thiourea derivatives may be appropriately selected in accordance with the intended purpose. Examples of the thiourea derivatives include ethylene thiourea, N-methylthiourea, N-ethylthiourea, N-propylthiourea, N-butylthiourea, N-laurylthiourea, N-phenylthiourea, N-cyclohexylthiourea, N,N-dimethylthiourea, N,N-diethylthiourea, N,N-dipropylthiourea, N,N-dibutylthiourea, N,N-dilaurylthiourea, N,N-diphenylthiourea, N,N-dicyclohexylthiourea, trimethylthiourea, tetramethylthiourea, N-acetylthiourea, N-benzoylthiourea, 1-allyl-3-(2-hydroxyethyl)-2-thiourea, 1-(2-tetrahydrofurfuryl)-2-thiourea, N-tert-butyl-N'-isopropylthiourea, 2-pyridylthiourea, and the like.

Of these, N-benzoylthiourea is preferable from the viewpoint of enhancing the curability of the dental adhesive composition.

These thiourea derivatives may be used alone or in combination.

No particular limitation is imposed on the amount of the thiourea derivative, and the amount of the thiourea derivative may be appropriately selected in accordance with the intended purpose. The amount of the thiourea derivative is preferably 0.1% by mass or more and 5% by mass or less, more preferably 0.1% by mass or more and 3% by mass or less, and further preferably 0.1% by mass or more and 1% by mass or less, relative to the total amount of the dental adhesive composition.

The amount of the thiourea derivative is preferably 0.1% by mass or more relative to the total amount of the dental adhesive composition because the curability of the dental adhesive composition is enhanced.

The amount of the thiourea derivative is preferably 5% by mass or less relative to the total amount of the dental adhesive composition because the solubility of the thiourea derivative in the radiopaque compound is increased.

### -Vanadium Compounds-

Of the chemical polymerization initiators, the vanadium compounds function as a reducing agent.

No particular limitation is imposed on the vanadium compounds, and the vanadium compounds may be appropriately selected in accordance with the intended purpose. Examples of the vanadium compounds include oxovanadium oxalate, vanadyl acetylacetonate, vanadium acetylacetonate, vanadyl stearate, vanadium naphthenate, vanadium benzoyl acetonate, and the like.

Of these, vanadyl acetylacetonate is preferable from the viewpoint of the curability of the dental adhesive composition.

These vanadium compounds may be used alone or in combination.

No particular limitation is imposed on the amount of the vanadium compound, and the amount of the vanadium compound may be appropriately selected in accordance with the intended purpose. The amount of the vanadium compound is preferably 0.001% by mass or more and 5% by mass or less, more preferably 0.0015% by mass or more and 1% by mass or less, and further preferably 0.002% by mass or more and 0.1% by mass or less, relative to the total amount of the dental adhesive composition.

The amount of the vanadium compound is preferably 0.001% by mass or more relative to the total amount of the dental adhesive composition because the curability of the dental adhesive composition is enhanced.

The amount of the vanadium compound is preferably 5% by mass or less relative to the total amount of the dental adhesive composition because the storage stability of the dental adhesive composition is improved.

### -Tertiary Amines-

Of the chemical polymerization initiators, the tertiary amines function as a reducing agent. The tertiary amines can also function as a photopolymerization accelerator.

No particular limitation is imposed on the tertiary amines, and the tertiary amines may be appropriately selected in accordance with the intended purpose. Examples of the tertiary amines include tertiary aliphatic amines, tertiary aromatic amines, and the like.

Examples of the tertiary aliphatic amines include N,N-dimethylaminoethyl methacrylate, triethanolamine, and the like.

Examples of the tertiary aromatic amines include alkyl p-dialkylaminobenzoate, 7-dimethylamino-4-methylcoumarin, N,N-dimethylaniline, N,N-dibenzylaniline, N,N-dimethyl-p-toluidine, N,N-diethyl-p-toluidine, N,N-bis(2-hydroxyethyl)-p-toluidine, N,N,2,4,6-pentamethylaniline, N,N,2,4-tetramethylaniline, N,N-diethyl-2,4,6-trimethylaniline, and the like.

Of these, the tertiary amine is preferably a tertiary aromatic amine, and more preferably alkyl p-dialkylaminobenzoate.

Examples of the alkyl p-dialkylaminobenzoate include methyl p-dimethylaminobenzoate, ethyl p-dimethylaminobenzoate, propyl p-dimethylaminobenzoate, amyl p-dimethylaminobenzoate, isoamyl p-dimethylaminobenzoate, ethyl p-diethylaminobenzoate, propyl p-diethylaminobenzoate, and the like.

These tertiary amines may be used alone or in combination.

### -Organic Peroxides-

Of the chemical polymerization initiators, the organic peroxides function as an oxidizing agent.

Examples of the organic peroxides include benzoyl peroxide, cumene hydroperoxide, t-butyl hydroperoxide, t-amyl hydroperoxide, 1,1,3,3-tetramethylbutyl hydroperoxide, 2,5-dimethyl-2,5-di(hydroperoxy)hexane, p-diisopropylbenzene monohydroperoxide, p-methane hydroperoxide, pinane hydroperoxide, and the like.

Of these, cumene hydroperoxide is preferable from the viewpoint of the curability of the dental adhesive composition.

These organic peroxides may be used alone or in combination.

No particular limitation is imposed on the amount of the organic peroxide, and the amount of the organic peroxide may be appropriately selected in accordance with the intended purpose. The amount of the organic peroxide is preferably 0.01% by mass or more and 10% by mass or less, more preferably 0.05% by mass or more and 5% by mass or less, and further preferably 0.1% by mass or more and 3% by mass or less, relative to the total amount of the dental adhesive composition.

The amount of the organic peroxide is preferably 0.01% by mass or more relative to the total amount of the dental adhesive composition because the curability of the dental adhesive composition is enhanced.

The amount of the organic peroxide is preferably 10% by mass or less relative to the total amount of the dental adhesive composition because the window during which the dental adhesive composition can be used is extended.

No particular limitation is imposed on the photopolymerization initiators, and the photopolymerization initiators may be appropriately selected in accordance with the intended purpose. Examples of the photopolymerization initiators include camphorquinone (CQ), ethyl 4-dimethylaminobenzoate (EPA), 2,4,6-trimethylbenzoyl diphenylphosphine oxide (TPO), phenylbis(2,4,6-trimethylbenzoyl)phosphine oxide, benzyl ketal, diacetyl ketal, benzyl dimethyl ketal, benzyl diethyl ketal, benzyl bis(2-methoxyethyl) ketal, 4,4'-dimethyl (benzyl dimethyl ketal), anthraquinone, 1-chloroanthraquinone, 2-chloroanthraquinone, 1,2-benzanthraquinone, 1-hydroxyanthraquinone, 1-methylanthraquinone, 2-ethylanthraquinone, 1-bromoanthraquinone, thioxanthone, 2-isopropylthioxanthone, 2-nitrothioxanthone, 2-methylthioxanthone, 2,4-dimethylthioxanthone, 2,4-diethylthioxanthone, 2,4-diisopropylthioxanthone, 2-chloro-7-trifluoromethylthioxanthone, thioxanthone-10,10-dioxide, thioxanthone-10-oxide, benzoin methyl ether, benzoin ethyl ether, benzoin isopropyl ether, benzoin isobutyl ether, benzophenone, bis(4-dimethylaminophenyl)ketone, 4,4'-bis(diethylamino)benzophenone, and the like.

Of these, camphorquinone (CQ), ethyl 4-dimethylaminobenzoate (EPA), and 2,4,6-trimethylbenzoyl diphenylphosphine oxide (TPO) are preferable from the viewpoint of enhancing the curability of the dental adhesive composition.

These photopolymerization initiators may be used alone or in combination.

No particular limitation is imposed on the amount of the photopolymerization initiator, and the amount of the photopolymerization initiator may be appropriately selected in accordance with the intended purpose. The amount of the photopolymerization initiator is preferably 0.01% by mass or more and 10% by mass or less, more preferably 0.05% by mass or more and 8% by mass or less, and more preferably 1% by mass or more and 5% by mass or less, relative to the total amount of the dental adhesive composition.

The amount of the photopolymerization initiator is preferably 0.01% by mass or more relative to the total amount of the dental adhesive composition because the curability of the dental adhesive composition is enhanced.

The amount of the photopolymerization initiator is preferably 10% by mass or less relative to the total amount of the dental adhesive composition because the window during which the dental adhesive composition can be used is extended.

### <<Polymerization Inhibitor>>

No particular limitation is imposed on the polymerization inhibitor, and the polymerization inhibitor may be appropriately selected in accordance with the intended purpose. Examples of the polymerization inhibitor include dibutylhydroxytoluene (2,6-di-tert-butyl-p-cresol) (BHT), 6-tert-butyl-2,4-xylenol, 1,4-dihydroxybenzene (HQ), 4-methoxyphenol (MEHQ), and the like.

Of these, dibutylhydroxytoluene (BHT) is preferable from the viewpoint of enhancing the curability of the dental adhesive composition.

These polymerization inhibitors may be used alone or in combination.

No particular limitation is imposed on the amount of the polymerization inhibitor, and the amount of the polymerization inhibitor may be appropriately selected in accordance with the intended purpose. The amount of the polymerization inhibitor is preferably 0.01% by mass or more and 5% by mass or less, more preferably 0.05% by mass or more and 3% by mass or less, and further preferably 0.1% by mass or more and 1% by mass or less, relative to the total amount of the dental adhesive composition.

The amount of the polymerization inhibitor is preferably 0.01% by mass or more and 5% by mass or less relative to the total amount of the dental adhesive composition because the storage stability of the dental adhesive composition is improved.

### <<Filler>>

No particular limitation is imposed on the filler, and the filler may be appropriately selected in accordance with the intended purpose. Examples of the filler include colloidal silica, surface-hydrophobized particulate silica (hydrophobized fumed silica), aluminum oxide, fluoroaluminosilicate glass, barium glass, strontium glass, and the like.

Of these, hydrophobized fumed silica (e.g., AEROSIL (registered trademark)) is preferable.

These fillers may be used alone or in combination.

No particular limitation is imposed on the amount of the filler, and the amount of the filler may be appropriately selected in accordance with the intended purpose. The amount of the filler is preferably 0.1% by mass or more and 30% by mass or less, more preferably 0.5% by mass or more and 20% by mass or less, and further preferably 1% by mass or more and 10% by mass or less, relative to the total amount of the dental adhesive composition.

The amount of the filler is preferably 0.1% by mass or more relative to the total amount of the dental adhesive composition because the viscosity of the dental adhesive composition increases, and thus the operability of the dental adhesive composition can be improved.

The amount of the filler is preferably 30% by mass or less relative to the total amount of the dental adhesive composition because the viscosity of the dental adhesive composition does not excessively increase, and thus the operability of the dental adhesive composition can be stabilized.

### <<Solvent>>

No particular limitation is imposed on the solvent, and the solvent may be appropriately selected in accordance with the intended purpose. Examples of the solvent include water, organic solvents, and the like.

No particular limitation is imposed on the organic solvents, and the organic solvents may be appropriately selected in accordance with the intended purpose. Examples of the organic solvents include ethanol, acetone, propanol, and the like.

These solvents may be used alone or in combination.

No particular limitation is imposed on the radiopacity in the cured product of the radiopaque compound, and the radiopacity may be appropriately selected in accordance with the intended purpose. The radiopacity in the cured product of the radiopaque compound is preferably 100% or more in terms of a ratio to aluminum.

No particular limitation is imposed on the evaluation method of the radiopacity in the cured product of the dental adhesive composition, and the evaluation method may be appropriately selected in accordance with the intended purpose. The radiopacity in the cured product of the dental adhesive composition can be measured by a radiopacity testing method for dental materials in accordance with ISO 13116, JIS T 6006, or the like.

### [Physical Properties of Dental Adhesive Composition]

No particular limitation is imposed on the physical properties of the dental adhesive composition, and the pH and viscosity are preferably as follows.

The pH of the dental adhesive composition is preferably 1 or higher and 10 or lower, and more preferably 1.5 or higher and 8 or lower. The dental adhesive composition having a pH in the above range exhibits less acidic or basic stimulation and excellent sensation upon use, and can stably maintain properties against environmental changes during long-term storage.

No particular limitation is imposed on the measurement method of the pH. The measurement method of the pH is, for example, a method in which 1 g of the dental adhesive composition is dissolved in 1 mL of an aqueous solvent, and the resulting solution is measured by a pH meter (e.g., HORIBA pH/ION METER F-72).

The viscosity of the dental adhesive composition is preferably 10 mPa·s or higher and 1,000 mPa·s or lower, and more preferably 50 mPa·s or higher and 500 mPa·s or lower. The dental adhesive composition having a viscosity in the above range exhibits excellent operability and high affinity to an adherend.

No particular limitation is imposed on the measurement method of the viscosity. For example, the viscosity can be measured by the following method.

### [Example of Measurement Method of Viscosity]

After a B-type viscometer (Toki Sangyo Co., Ltd.) is allowed to be constant at 23°C, 0.6 mL of the dental adhesive composition is weighed and allowed to stand for 5 minutes to be constant in temperature. The measurement is performed at 10 rpm for 5 minutes, and the viscosity is the average value from passage of 2 minutes to passage of 5 minutes.

No particular limitation is imposed on the production method of the dental adhesive composition. For example, the dental adhesive composition can be produced by mixing and stirring the above components.

No particular limitation is imposed on the use of the dental adhesive composition. For example, the dental adhesive composition can be used for various dental materials.

Examples of the dental materials include dental cements, dental adhesives, dental temporary sealants, dental primers, dental coatings, dental filling composite resins, dental hard resins, dental cutting resins, dental temporary restoration materials, dental fillers, dentifrices, and the like.

Of these, the dental adhesive composition is preferably used for dental adhesives, dental filling composite resins, and dental cements.

### EXAMPLES

Hereinafter, the present invention will be further described by way of Examples. In the following, a numerical value without units, "parts", or "%" is on a mass basis unless otherwise specified.

### <Synthesis of Compounds 1 to 5>

### <<Synthesis of Compound 1>>

Trans-2,3-dibromo-2-butene-1,4-diol (obtained from TOKYO CHEMICAL INDUSTRY CO., LTD.), mono(2-acryloyloxyethyl) succinate (obtained from TOKYO CHEMICAL INDUSTRY CO., LTD.), and a solution were heated overnight under reflux. At this time, a catalyst and a condensing agent were also added, and synthesis was performed in a protective gas atmosphere. Next, the reaction product was cooled to room temperature. Subsequently, the reaction product was extracted with a solution, followed by washing with water at least once and then drying over anhydrous Na₂SO₄. The solvent was removed in vacuum to obtain Compound 1.

The structure of trans-2,3-dibromo-2-butene-1,4-diol is as follows.

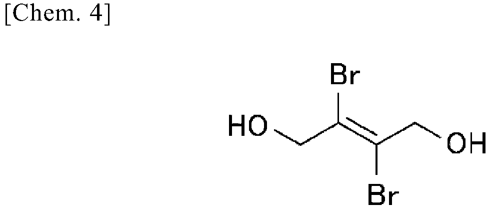

The structure of mono(2-acryloyloxyethyl) succinate is as follows.

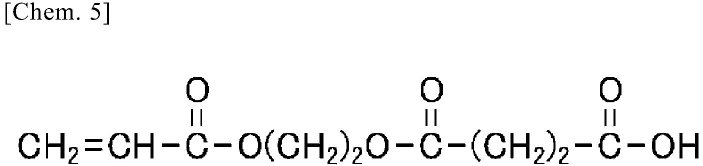

### <<Synthesis of Compound 2>>

Compound 2 was obtained by the same method as the method for the synthesis of Compound 1, except that 2-carboxyethyl acrylate (obtained from TOKYO CHEMICAL INDUSTRY CO., LTD.) was used instead of mono(2-acryloyloxyethyl) succinate. The structure of 2-carboxyethyl acrylate is as follows.

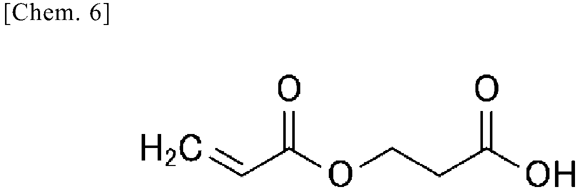

### <<Synthesis of Compound 3>>

Compound 3 was obtained by the same method as the method for the synthesis of Compound 1, except that 2,2-bis(bromomethyl)-1,3-propanediol (obtained from TOKYO CHEMICAL INDUSTRY CO., LTD.) was used instead of trans-2,3-dibromo-2-butene-1,4-diol. The structure of 2,2-bis(bromomethyl)-1,3-propanediol is as follows.

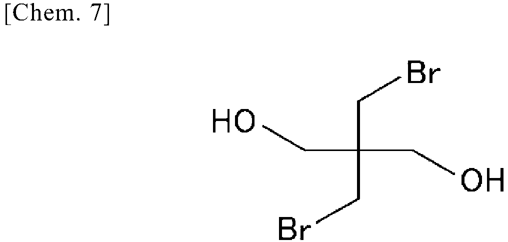

### <<Synthesis of Compound 4>>

Compound 4 was obtained by the same method as the method for the synthesis of Compound 1, except that trans-2 butene-1,4-diol (obtained from TOKYO CHEMICAL INDUSTRY CO., LTD.) was used instead of trans-2,3-dibromo-2-butene-1,4-diol. The structure of trans-2 butene-1,4-diol is as follows.

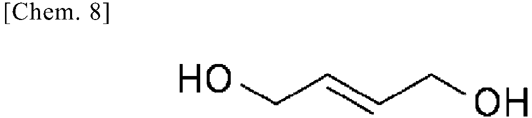

### <<Synthesis of Compound 5>>

Compound 5 was obtained by the same method as the method for the synthesis of Compound 1, except that 2,5-dibromo-1,4-benzenediol (obtained from TOKYO CHEMICAL INDUSTRY CO., LTD.) was used instead of trans-2,3-dibromo-2-butene-1,4-diol. The structure of 2,5-dibromo-1,4-benzenediol is as follows.

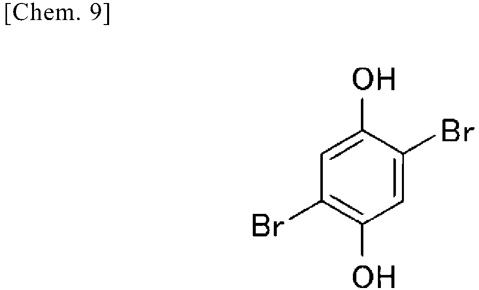

### (Examples 1 to 9 and Comparative Examples 1 to 3)

### <Preparation of Dental Adhesive Compositions>

Dental adhesive compositions (Examples 1 to 9 and Comparative Examples 1 to 3) having the compositions shown in Tables 1 and 2 were prepared. Each of the dental adhesive compositions was evaluated for radiopacity, halogen release, adhesion strength to ceramics, and adhesion strength to dentin by the following methods. The results are shown in Tables 1 and 2.

### <Evaluation of Radiopacity>

When the dental adhesive composition is actually used, the dental adhesive composition is applied to an adherend, and then dried in air to remove the solvent, followed by photocuring. Considering this, the dental adhesive compositions of Examples 1 to 9 and Comparative Examples 1 to 3, from which the solvent components were removed, were used for the evaluation of radiopacity.

An OHP sheet was laid on a glass slide, and a ring having a diameter of 1.5 cm and a thickness of 1.0 mm was placed. The ring was charged with each of the dental adhesive compositions from which the solvents were removed, and then an OHP sheet and a glass slide were sequentially placed thereon to prepare an evaluation sample. Each of the front and rear surfaces of the evaluation sample were irradiated at 9 points in a 10-second mode using a light irradiator (G-Light Prima-II plus, obtained from GC CORPORATION). The obtained cured material was subjected to cephalography using an X-ray imaging device (Aadva GX-100 2D, obtained from GC CORPORATION) with an aluminum step. The obtained image was analyzed by image J, and the radiopacity (%) to aluminum was calculated. The evaluation criteria of the radiopacity were as follows.

### -Evaluation Criteria-

A: The radiopacity to aluminum is 100% or more.
B: The radiopacity to aluminum is 50% or more and less than 100%.
C: The radiopacity to aluminum is less than 50%.

### <Evaluation of Halogen Release>

Each dental adhesive composition was dropped onto a dish one drop by one drop, and then allowed to stand for 10 minutes under natural light. Subsequently, the color change of the dental adhesive composition was visually observed.

In the dental adhesive compositions of Examples 1 to 9 and Comparative Examples 1 and 3, no color change of the compositions was visually observed. That is, the color change of only the dental adhesive composition of Comparative Example 2 was visually observed. The color change of the dental adhesive composition is presumed to be due to halogen release in the dental adhesive composition caused by natural light.

### <Evaluation of Adhesion Strength to Enamel and Adhesion Strength to Dentin>

The tooth substance was abraded with a #400 water-resistant abrasive paper sheet, and a fresh surface was exposed. Each dental adhesive composition was applied and dried immediately by air blow. A plastic mold having a hole 2.38 mm in diameter was placed on a test surface, and light irradiation was performed from a position above the mold for 10 seconds using a light irradiator (G-Light Prima-II plus, obtained from GC CORPORATION). Next, composite resins were charged, and light irradiation was performed for 20 seconds. After immersion in water of 37°C for 24 hours, an autograph (EZ-S, SHIMADZU) was used to perform a shear test on five test samples at a crosshead speed of 1 mm/min. The average value of the shear adhesion strengths was determined to evaluate the adhesiveness of each composite resin to the tooth substance.

The evaluation criteria of the adhesiveness of the composite resin to the tooth substance (enamel, dentin) at the time of using each dental adhesive composition are as follows.

### -Evaluation Criteria-

A: The average shear adhesion strength is 15 MPa or more.
B: The average shear adhesion strength is less than 15 MPa.

**[Table 1]**

| | | **Examples** | | | | | | | | |
|---|---|---|---|---|---|---|---|---|---|---|
| | | **1** | **2** | **3** | **4** | **5** | **6** | **7** | **8** | **9** |
| **Compound 1** | | 20.0 | 8.9 | 9.0 | - | - | - | - | - | - |
| **Compound 2** | | - | - | - | 18.0 | 7.2 | 9.1 | - | - | - |
| **Compound 3** | | - | - | - | - | - | - | 21.1 | 10.0 | 11.5 |
| **Compound 4** | | - | - | - | - | - | - | - | - | - |
| **Compound 5** | | - | - | - | - | - | - | - | - | - |
| **Acid groupcontaining methacrylate** | **4-META** | 11 | 7 | 12 | 6 | 7 | 11 | 13 | 12 | 7 |
| | **MDP** | 6 | 11 | 8 | 6 | 13 | 10 | 7 | 6 | 12 |
| **Acid group-free methacrylate** | **GDMA** | - | 11 | 8 | - | - | 7 | - | 10 | 3 |
| | **TEGDMA** | - | - | 2 | - | 13 | 4 | - | 3 | 6 |
| **Polymerization initiator** | **TPO** | 2 | 2 | 2 | 2 | 2 | 2 | 2 | 2 | 2 |
| | **CQ** | 1.0 | 1.8 | 2.0 | 0.8 | 1.9 | 2.0 | 1.0 | 1.2 | 1.6 |
| | **EPA** | 1 | 1 | 1 | 1 | 1 | 1 | 1 | 1 | 1 |
| **Polymerization inhibitor** | **BHT** | 1 | 1 | 1 | 1 | 1 | 1 | 1 | 1 | 1 |
| **AEROSIL** | **R972** | 5 | 5 | 5 | 5 | 5 | 5 | 5 | 5 | 5 |
| **Organic solvent** | **Acetone** | 28.0 | 31.3 | 32.0 | 39.2 | 33.9 | 32.9 | 27.9 | 27.8 | 34.9 |
| **Water** | **Distilled water** | 25 | 20 | 18 | 21 | 15 | 15 | 21 | 21 | 15 |
| **Total** | | 100 | 100 | 100 | 100 | 100 | 100 | 100 | 100 | 100 |
| | | | | | | | | | | |
| **Evaluation results** | **Radiopacity to AI (%)** | A 121 | B 58 | B 63 | A 113 | B 72 | B 69 | A 112 | B 52 | B 59 |
| | **Browning after 10 minutes** | None | None | None | None | None | None | None | None | None |
| | **Adhesion strength to enamel [Mpa]** | A 23.3 | A 26.2 | A 27.3 | A 22.6 | A26.7 | A 28.1 | A 24.1 | A 24.5 | A 26.8 |
| | **Adhesion strength to dentin [Mpa]** | B 14.2 | A 21.3 | A 23.6 | A 15.2 | A 25.7 | A28.5 | A 17.2 | A 25.1 | A 24.8 |

**[Table 2]**

| | | **Comparative Examples** | | |
|---|---|---|---|---|
| | | **1** | **2** | **3** |
| **Compound 1** | | - | - | - |
| **Compound 2** | | - | - | - |
| **Compound 3** | | - | - | - |
| **Compound 4** | | 18.2 | - | - |
| **Compound 5** | | - | 16.4 | - |
| **Acid groupcontaining methacrylate** | **4-META** | 14 | 6 | 8 |
| | **MDP** | 9 | 6 | 8 |
| **Acid group-free methacrylate** | **GDMA** | - | - | 10 |
| | **TEGDMA** | - | - | 5 |
| **Polymerization initiator** | **TPO** | 2 | 2 | 2 |
| | **CQ** | 1.5 | 2.0 | 1.6 |
| | **EPA** | 1 | 1 | 1 |
| **Polymerization inhibitor** | **BHT** | 1 | 1 | 1 |
| **AEROSIL** | **R972** | 5 | 5 | 5 |
| **Organic solvent** | **Acetone** | 28.3 | 45.6 | 35.4 |
| **Water** | **Distilled water** | 20 | 15 | 23 |
| **Total** | | 100 | 100 | 100 |
| | | | | |
| **Evaluation results** | **Radiopacity to Al (%)** | C 0 | A 102 | C 0 |
| | **Browning after 10 minutes** | None | Present | None |
| | **Adhesion strength to enamel [Mpa]** | A 24.2 | A 21.5 | A 26.7 |
| | **Adhesion strength to dentin [Mpa]** | A 29.6 | B 13.8 | A 30.1 |

Embodiments of the present invention are, for example, as follows.
<1>
   A radiopaque compound, including:
   a structural moiety A including a halogen atom and having an acyclic structure; and
   a structural moiety B derived from a (meth)acrylate.
<2>
   The radiopaque compound according to <1> above, wherein
   the radiopaque compound includes at least one structure selected from a structure represented by general formula (1) below, a structure represented by general formula (2) below, or a structure represented by general formula (3) below: 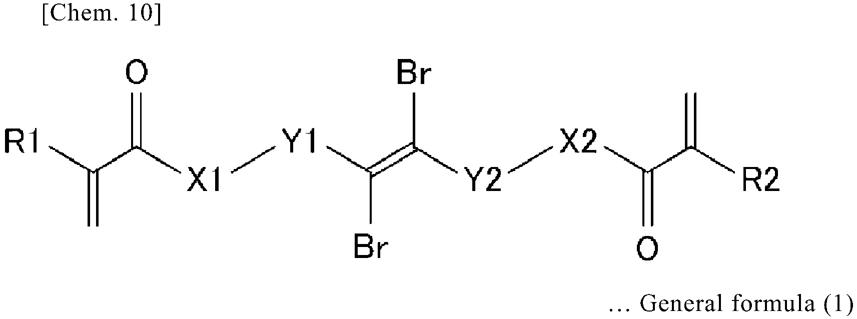 (in the general formula (1), R1 and R2 each independently represent "-H" or "-CH₃", X1 and X2 each independently represent "-O-" or "-NH-", and Y1 and Y2 each independently represent a linear or branched hydrocarbon group having 1 or more and 20 or less carbon atoms, which optionally includes an ether bond, an ester bond, and/or an amide bond); 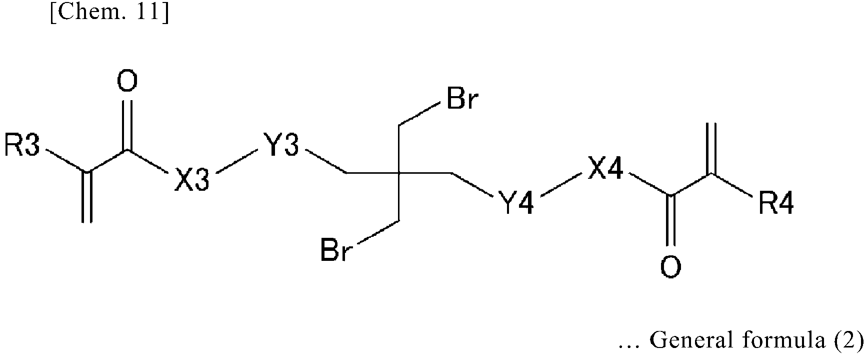 (in the general formula (2), R3 and R4 each independently represent "-H" or "-CH₃", X3 and X4 each independently represent "-O-" or "-NH-", and Y3 and Y4 each independently represent a linear or branched hydrocarbon group having 1 or more and 20 or less carbon atoms, which optionally includes an ether bond, an ester bond, and/or an amide bond); and 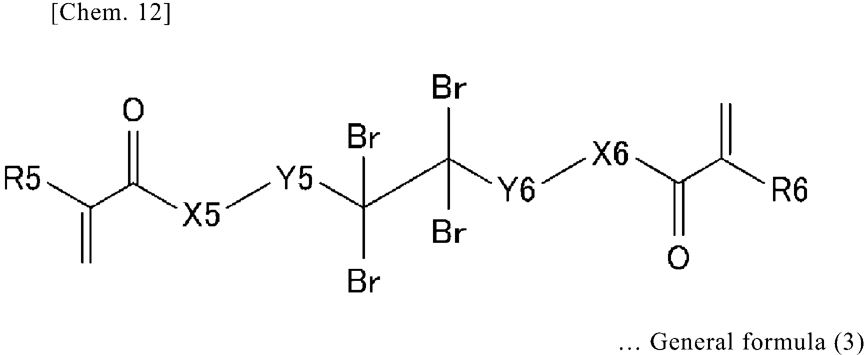 (in the general formula (3), R5 and R6 each independently represent "-H" or "-CH₃", X5 and X6 each independently represent "-O-" or "-NH-", and Y5 and Y6 each independently represent a linear or branched hydrocarbon group having 1 or more and 20 or less carbon atoms, which optionally includes an ether bond, an ester bond, and/or an amide bond).
<3>
   A dental adhesive composition, including:
   a radiopaque compound, wherein
   the radiopaque compound includes
      a structural moiety A including a halogen atom and having an acyclic structure; and
      a structural moiety B derived from a (meth)acrylate.
<4>
   The dental adhesive composition according to <3> above, wherein
   the radiopaque compound includes at least one structure selected structure from a structure represented by general formula (1) below, a structure represented by general formula (2) below, or a structure represented by general formula (3) below: 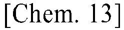 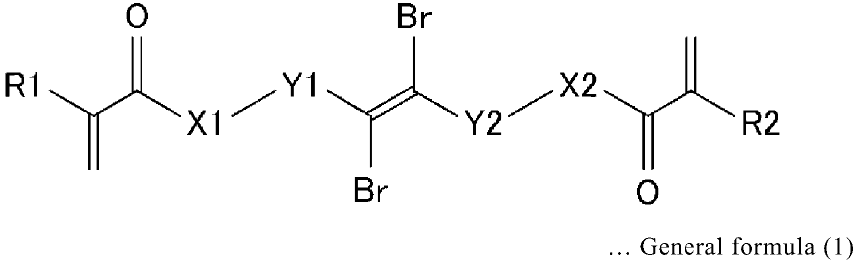 (in the general formula (1), R1 and R2 each independently represent "-H" or "-CH₃", X1 and X2 each independently represent "-O-" or "-NH-", and Y1 and Y2 each independently represent a linear or branched hydrocarbon group having 1 or more and 20 or less carbon atoms, which optionally includes an ether bond, an ester bond, and/or an amide bond); 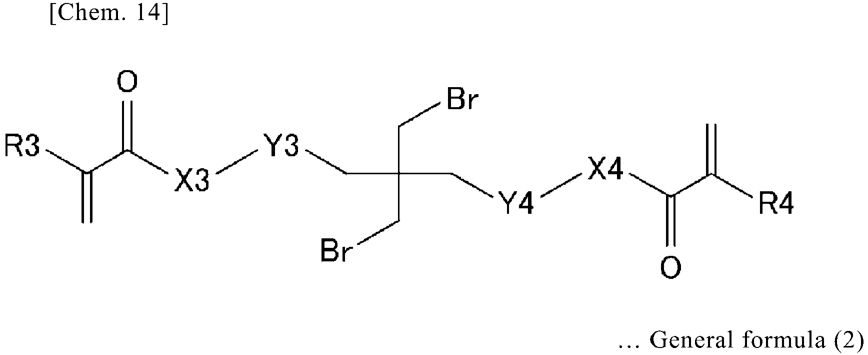 (in the general formula (2), R3 and R4 each independently represent "-H" or "-CH₃", X3 and X4 each independently represent "-O-" or "-NH-", and Y3 and Y4 each independently represent a linear or branched hydrocarbon group having 1 or more and 20 or less carbon atoms, which optionally includes an ether bond, an ester bond, and/or an amide bond); and 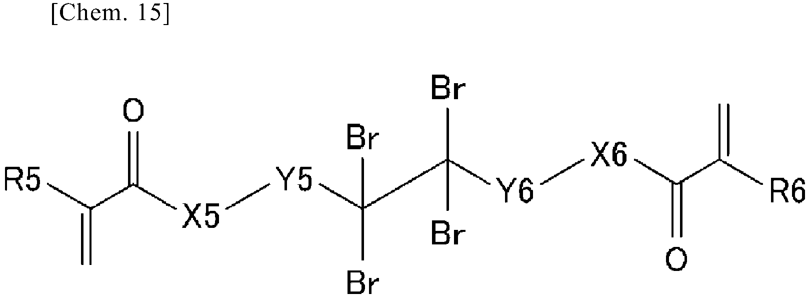 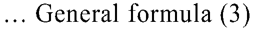 (in the general formula (3), R5 and R6 each independently represent "-H" or "-CH₃", X5 and X6 each independently represent "-O-" or "-NH-", and Y5 and Y6 each independently represent a linear or branched hydrocarbon group having 1 or more and 20 or less carbon atoms, which optionally includes an ether bond, an ester bond, and/or an amide bond).
<5>
   The dental adhesive composition according to <3> or <4> above, wherein
   the dental adhesive composition includes a photopolymerization initiator.
<6>
   The dental adhesive composition according to any one of <3> to <5> above, wherein
   the dental adhesive composition includes water.
<7>
   The dental adhesive composition according to any one of <3> to <6> above, wherein
   radiopacity in a cured product of the dental adhesive composition is 100% or more in terms of a ratio to aluminum.
<8>
   The dental adhesive composition according to any one of <3> to <7> above, wherein
   an amount of the radiopaque compound is 0.5% by mass or more and 70% by mass or less relative to a total amount of the dental adhesive composition.

According to the radiopaque compound according to <1> or <2> and the dental adhesive composition according to any one of <3> to <8>, various conventional problems can be solved, and the object of the present invention can be achieved.

This application claims priority to Japanese Patent Application No. 2023-144346, filed on September 6, 2023, the entire contents of which are incorporated herein by reference.

## Claims

1. A radiopaque compound, comprising:
a structural moiety A including a halogen atom and having an acyclic structure; and
a structural moiety B derived from a (meth)acrylate.

2. The radiopaque compound according to claim 1, wherein
the radiopaque compound includes at least one structure selected from a structure represented by general formula (1) below, a structure represented by general formula (2) below, or a structure represented by general formula (3) below: 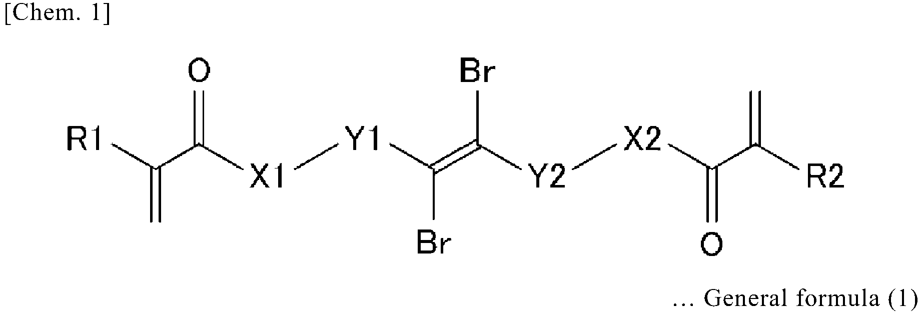 (in the general formula (1), R1 and R2 each independently represent "-H" or "-CH₃", X1 and X2 each independently represent "-O-" or "-NH-", and Y1 and Y2 each independently represent a linear or branched hydrocarbon group having 1 or more and 20 or less carbon atoms, which optionally includes an ether bond, an ester bond, and/or an amide bond); 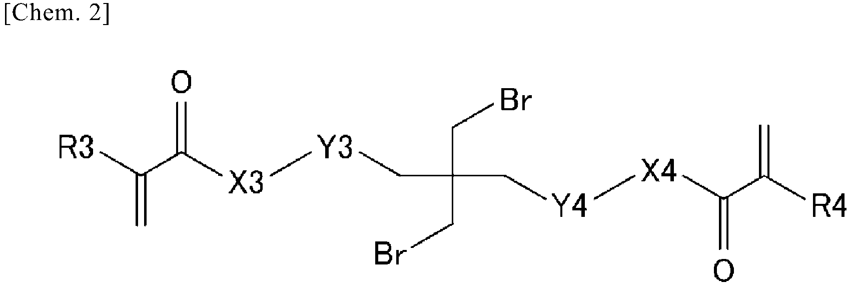 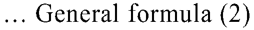 (in the general formula (2), R3 and R4 each independently represent "-H" or "-CH₃", X3 and X4 each independently represent "-O-" or "-NH-", and Y3 and Y4 each independently represent a linear or branched hydrocarbon group having 1 or more and 20 or less carbon atoms, which optionally includes an ether bond, an ester bond, and/or an amide bond); and 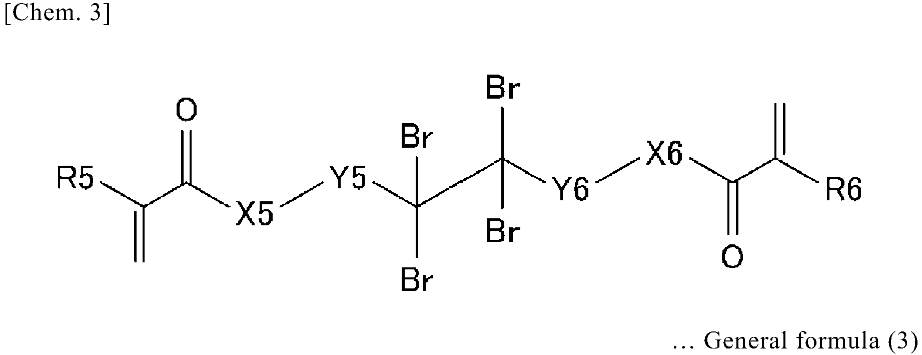 (in the general formula (3), R5 and R6 each independently represent "-H" or "-CH₃", X5 and X6 each independently represent "-O-" or "-NH-", and Y5 and Y6 each independently represent a linear or branched hydrocarbon group having 1 or more and 20 or less carbon atoms, which optionally includes an ether bond, an ester bond, and/or an amide bond).

3. A dental adhesive composition, comprising:
a radiopaque compound, wherein
the radiopaque compound includes
a structural moiety A including a halogen atom and having an acyclic structure; and
a structural moiety B derived from a (meth)acrylate.

4. The dental adhesive composition according to claim 3, wherein
the radiopaque compound includes at least one structure selected from a structure represented by general formula (1) below, a structure represented by general formula (2) below, or a structure represented by general formula (3) below: 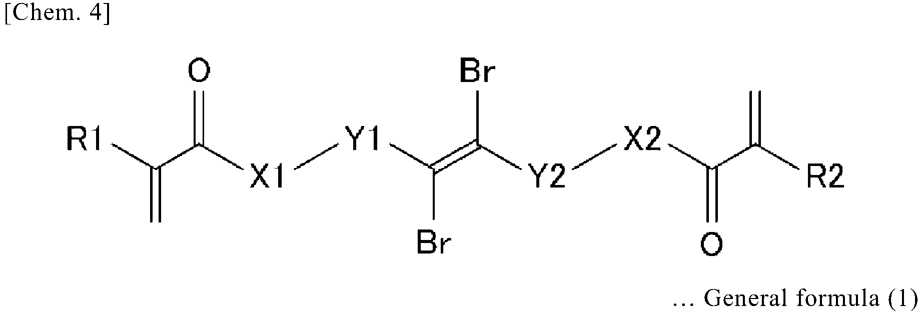 (in the general formula (1), R1 and R2 each independently represent "-H" or "-CH₃", X1 and X2 each independently represent "-O-" or "-NH-", and Y1 and Y2 each independently represent a linear or branched hydrocarbon group having 1 or more and 20 or less carbon atoms, which optionally includes an ether bond, an ester bond, and/or an amide bond); 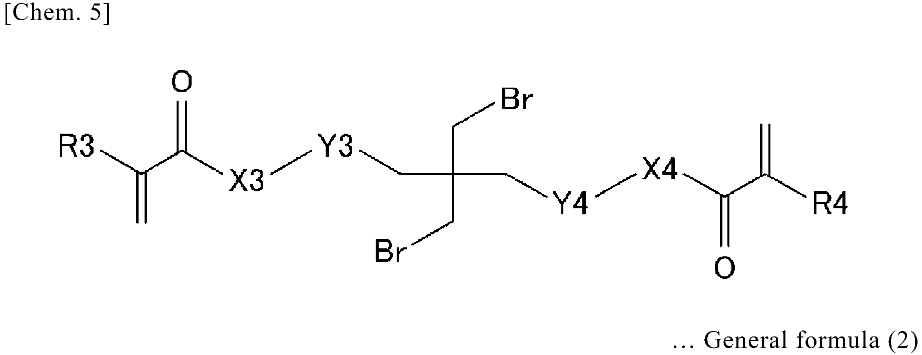 (in the general formula (2), R3 and R4 each independently represent "-H" or "-CH₃", X3 and X4 each independently represent "-O-" or "-NH-", and Y3 and Y4 each independently represent a linear or branched hydrocarbon group having 1 or more and 20 or less carbon atoms, which optionally includes an ether bond, an ester bond, and/or an amide bond); and 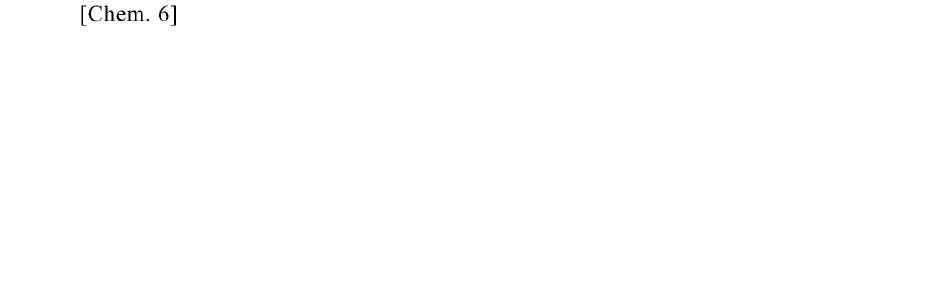 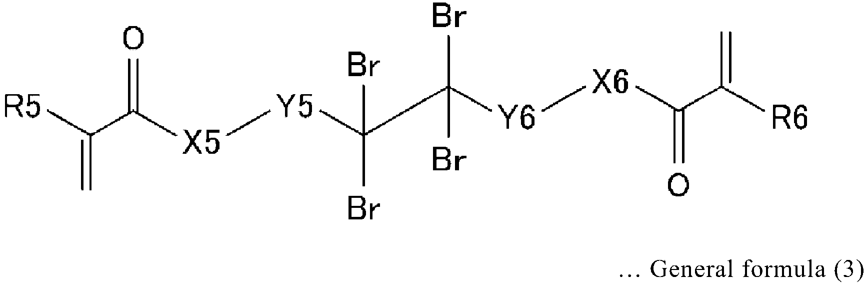 (in the general formula (3), R5 and R6 each independently represent "-H" or "-CH₃", X5 and X6 each independently represent "-O-" or "-NH-", and Y5 and Y6 each independently represent a linear or branched hydrocarbon group having 1 or more and 20 or less carbon atoms, which optionally includes an ether bond, an ester bond, and/or an amide bond).

5. The dental adhesive composition according to claim 3 or 4, wherein
the dental adhesive composition includes a photopolymerization initiator.

6. The dental adhesive composition according to any one of claims 3 to 5, wherein
the dental adhesive composition includes water.

7. The dental adhesive composition according to any one of claims 3 to 6, wherein
radiopacity in a cured product of the dental adhesive composition is 100% or more in terms of a ratio to aluminum.

8. The dental adhesive composition according to any one of claims 3 to 7, wherein
an amount of the radiopaque compound is 0.5% by mass or more and 70% by mass or less relative to a total amount of the dental adhesive composition.
